# EUROPEAN PATENT SPECIFICATION

(11) **EP 3 986 512 B1**
(45) Date of publication and mention of the grant of the patent: **25.11.2026**
(21) Application number: 20733719.7
(22) Date of filing: 15.06.2020
(51) Int. Cl.: A61M 60/13, A61M 60/174, A61M 60/216, A61M 60/414, A61M 60/808, A61M 60/829, A61M 60/867, A61M 25/00, A61M 25/10, A61M 5/31, A61M 5/36, G01C 9/00, A61M 1/36

(54) **SYSTEM AND METHOD FOR PREPARING A CATHETER BEFORE USE**
SYSTEM UND VERFAHREN ZUR VORBEREITUNG EINES KATHETERS VOR GEBRAUCH
SYSTÈME ET PROCÉDÉ DE PRÉPARATION D'UN CATHÉTER AVANT L'USAGE

(30) Priority: 18.06.2019 EP 19180927
(43) Date of publication of application: 27.04.2022
(73) Proprietor: Abiomed Europe GmbH, 52074 Aachen (DE)
(72) Inventor: VISSER, Johannes, 52074 Aachen (DE); CHUEV, Andre, 52074 Aachen (DE)
(74) Representative: Klunker IP Patentanwälte PartG mbB
(86) International application number: PCT/EP2020/066459
(87) International publication number: WO 2020/254233

(56) References cited:
- US-A1- 2012 178 985
- US-A1- 2018 168 726
- US-B2- 7 682 345

## Description

### FIELD OF THE INVENTION

This invention relates to a method of preparing a catheter for use in a patient, in particular purging and de-airing, and further relates to a respective system including the catheter. The catheter may be part of an intravascular blood pump for percutaneous insertion into a patient's blood vessel.

### BACKGROUND OF THE INVENTION

Before using a catheter in a patient, more specifically before inserting the catheter into a patient's blood vessel, the catheter must be properly prepared. In particular, the catheter must be purged and de-aired to prevent air or other gas bubbles from being introduced into the patient, which could cause severe complications, such as an infarct. Typically, a catheter comprises an elongate tubular body having a proximal end and a distal end, with the distal end being the leading end upon insertion into the patient. The tubular body has a lumen which extends through the catheter from the proximal end to the distal end for receiving functional structures, lines or the like, depending on the application or for supplying fluids, e.g. including pharmaceuticals, to the patient. The lumen includes air before use and shall be completely de-aired before the catheter is inserted into the patient. For de-airing and purging, a fluid may be pumped through the catheter with a certain pressure.

De-airing the lumen of the elongate tubular body of the catheter usually is not a problem because of its smooth geometry and small diameter. The lumen typically does not have any convexities, and the small diameter causes capillary forces that draw the purge fluid through the lumen to eliminate any air bubbles or other gas bubbles. However, the catheter may be in fluid communication to at least one connected device, e.g. a pump unit or a handle portion, which can be held by a user to maneuver or otherwise control the function of the catheter. Depending on the application, a connected device may have at least one cavity which is in fluid communication with the lumen of the catheter and which has a fluid inlet for receiving the purge fluid. The cavity of a connected device may have a complex geometry and may accommodate functional and possibly movable parts, such that air bubbles may still be trapped in the cavity although the purge fluid already exits the catheter at the distal end. In particular, if a user does not correctly hold the connected device, for instance not in a correct de-airing orientation which may be on an upright and vertical orientation, air bubbles may be trapped inside the cavity. According to the state of the art, it highly depends on the experience of the user, who may be for instance a surgeon, cardiologist, general practitioner, or other medical staff, to determine when the catheter and connected devices are completely de-aired.

The aforementioned problem of de-airing the catheter, in particular a connected device, may be particularly relevant to intravascular blood pumps. An intravascular blood pump is configured for percutaneous insertion into a patient's blood vessel and comprises a pump unit with a rotatable impeller for conveying blood from a blood flow inlet to a blood flow outlet of the pump unit. A drive unit is provided to cause rotation of the impeller.

In one type of intravascular blood pumps the pump unit comprises a drive unit which is directly coupled to the impeller and is included in a common pump casing together with the pump unit. The common pump casing may be arranged at the distal end of the catheter. De-airing of the catheter may be required before insertion into a patient.

In another type of intravascular blood pumps, the drive unit may be coupled to the impeller by means of a flexible drive shaft which extends through the lumen of the elongate tubular portion of the catheter, wherein the drive unit may be disposed in a cavity of a handle portion. In this type of intravascular blood pump, the pump unit with the impeller may be expandable. The drive unit may comprise an electric motor including a stator and a rotor and further electronic parts to control rotation of the drive unit, which may create convexities, undercuts or other cavities in which air bubbles might be trapped. Since the drive unit, such as the moving parts of an electric motor, may be immersed in the purge fluid, it is even more difficult to properly eliminate all air bubbles from the cavity in the handle portion. Thus, it is important that a user holds the catheter, in particular the handle portion, in a de-airing orientation during purging and de-airing to avoid causing harm to the patient.
US 2018/168726 A1 discloses that cardiac catheterization is conducted using a probe having a balloon assembly, in which a spring has a resting axially elongated configuration and a torsed, axially shortened configuration. A balloon is twisted about the spring and mechanically linked to the spring. A flexible sheath is disposed about the spring within the balloon, the sheath and the wall of the balloon defining a chamber to contain a fluid flowing therein and thereout of the balloon.
US 7,682,345 B2 discloses a medical injector system for injecting fluid from a syringe into a patient, the syringe having a barrel and a plunger, the plunger comprising an inner surface having an engaging portion adapted to be releasably engaged by a plunger handle which has a retention member adapted to releasably engage the engaging portion of the plunger.

### SUMMARY OF THE INVENTION

It is therefore an object of the present invention to provide a method of preparing, in particular purging, a catheter for use in a patient and a respective system including the catheter, which allows securely de-airing the catheter, particularly before insertion into a patient. In particular, the catheter may be part of an intravascular blood pump.

This object is achieved according to the present invention by a system and a method having the features of the independent claims. Preferred embodiments and further developments of the invention are specified in the claims dependent thereon.

According to a first aspect of the invention, a system is provided which comprises a catheter as described above, in particular comprising an elongated tubular portion and a connected device which is connected to the catheter. The elongate tubular portion has a lumen. The connected device has at least one cavity. As explained above, before the catheter is used in a patient, the lumen and the cavity should be de-aired, i.e. air bubbles should be completely removed. In order to monitor the orientation of at least one connected device and for the elongate tubular portion, preferably a connected device as is described in more detail below, the system further comprises at least one sensor for detecting said orientation. The system according to the invention comprises all technical features defined in claim 1.

According to a second aspect of the invention, a method of preparing a catheter for use in the patient is provided. In particular, the catheter is constructed as explained above having an elongated tubular portion and a connected device. In the method of preparing the catheter, in particular purging the catheter, a fluid is supplied through a fluid inlet into the at least one cavity of the connected device and thereby into the lumen of the elongate tubular portion to purge said cavity and lumen. The method further comprises the step of detecting the orientation of at least one connected device and of the elongate tubular portion, preferably during the fluid supply. The method according to the invention comprises all steps defined in claim 7.

In the system and method of the present invention, an orientation of at least one connected device and/or the elongate tubular portion, preferably a connected device, is detected. Thus, the system and the method of the present invention do not rely on the experience of the user to correctly orient the catheter, especially of a connected device, but provide means to monitor the orientation of at least a portion of the catheter, in particular during the purging process. It will be appreciated that the term "connected device" may refer to any structure connected to the elongate tubular body which can be manipulated by a user, in particular holding it by a user in his hands or turning it together with the patient, and which has a cavity in fluid communication with the lumen of the elongate tubular body.

The term "orientation" particularly refers to the orientation of the respective portion of the catheter in three-dimensional space relative to a fixed reference system such as gravity. In particular, the orientation does not necessarily include an absolute position of the respective portion of the catheter in three-dimensional space, but may rather be regarded as a "relative position", for instance relative to a vertical or horizontal axis or plane in three-dimensional space or any other suitable reference point or system. For instance, the orientation may refer to a tilt of the respective portion of the catheter and may be indicated as an angle in degrees, e.g. relative to a vertical axis or horizontal plane. A vertical axis can be oriented in direction of gravity.

A control unit is provided, which receives data from the at least one sensor, the data particularly including the detected orientation, i.e. data representing the actual or current orientation of the respective portion of the catheter. The data may be transmitted by wire or wirelessly. It is determined whether the detected orientation matches an orientation in which a cavity can be de-aired. This may be the cavity of which the orientation is detected. A difference between the detected orientation and a predetermined orientation, i.e. a predefined orientation which should be achieved by a user orienting the catheter, is calculated. The predetermined
target orientation is not necessarily a unique orientation but may include a range of orientations suitable for proper purging as will be explained in more detail below.

Based on said difference between the detected orientation and the predetermined orientation, a user may be guided to change, i.e. to correct, the orientation of the respective portion of the catheter to approach the predetermined orientation. The respective portion of the catheter can be moved such that it matches the predetermined orientation or at least lies within a range defined by the predetermined orientation. It will be appreciated that a tolerance may be allowed. For instance, a user interface, such as a display or the like, may be provided in the control unit, which may display the detected orientation or a representation indicating the difference between the detected orientation and the predetermined orientation or both. Alternatively or additionally, it may display the matching of the detected orientation with an orientation in which a cavity may be de-aired, which may be a predetermined orientation. For instance, an image of the respective portion of the catheter indicating its orientation in real time may be displayed, or a simple graphic, such as a line or the like, may be displayed indicating the tilt angle. Additionally or alternatively, the difference between the detected orientation and the predetermined target orientation can be displayed. For instance, any suitable graphic, such as a symbol, color scale (e.g. red-yellow-green) or the like may be displayed. Alternatively or additionally, the difference may also be indicated by means of any suitable acoustic signal, which enables the user to change the orientation to approach the predetermined target orientation.

Guiding the user to correct the orientation of the respective portion of the catheter can improve the purging process and can reduce or eliminate the risk of air bubbles being trapped inside the cavity or lumen. In particular, a complex geometry inside the cavity or lumen, e.g. comprising convexities, undercuts or the like, may require the user to hold the respective portion of the catheter in the predetermined orientation such that air bubbles can exit.

Since air bubbles move in the purge fluid in a vertically upward direction, the predetermined orientation is preferably a vertical or substantially vertical orientation or a conical range defined by a vertical axis and a predetermined angle. It will be appreciated that any other orientation may be possible, depending on the geometry of the cavity or lumen to be de-aired. For instance, depending on the interior geometry, an angle of 45 degrees or any other angle could be necessary or suitable to completely de-air the cavity. As mentioned above, the "predetermined orientation" may include a range of orientations, e.g. range of angles, within which it is possible to completely de-air the cavity and lumen of the catheter. For instance, if the cavity has a smooth and regular geometry, it may be sufficient if the user holds the respective portion of the catheter, in particular the connected device, within a range of 45 or 30 degrees in any direction with respect to a vertical axis. Preferably, the orientation can be detected in the range of 0 to 180 degrees, e.g. to determine which end of the handle portion faces upward and which end faces downward. Generally, in order to ensure proper de-airing, the fluid inlet of the handle portion should be located below an exit to the lumen of the elongate tubular portion, with respect to a vertical direction. For example, an orientation angle can refer to a longitudinal main direction of the catheter or a connected device.

It will be appreciated that the predetermined orientation, regardless of whether it refers to a single orientation or a range of orientations, may not only refer to the tilt, i.e. angle relative to an external reference system, of the respective portion of the catheter, but may also refer to any other degree of freedom, such as rotation about a longitudinal axis of the respective portion of the catheter. For instance, in particular if the predetermined orientation is other than a vertical orientation, it might be necessary to rotate e.g. the connected device about its longitudinal axis to a predetermined orientation to remove all air bubbles from the cavity.

In one embodiment, a user may start the purge process and a fluid, i.e. any suitable purge fluid, will be supplied to the cavity of the connected device and thereby into the lumen of the tubular portion of the catheter. The purge fluid may be continuously supplied to the cavity and lumen of the catheter while the user is continuously guided to hold the respective portion of the catheter in the correct orientation, i.e. to change the orientation if necessary.

In a preferred embodiment, however, the fluid supply may be activated, in particularly activated only, if the detected orientation matches the predetermined target orientation, i.e. if the user correctly orients the catheter and/or a connected device the respective portion of the catheter. Similarly, the fluid supply may be deactivated, i.e. stopped or paused, if the detected orientation differs from the predetermined target orientation, i.e. when the user does not correctly orient the respective portion of the catheter. Particularly in this embodiment, it may be advantageous to measure the volume of the supplied fluid. Thus, if the measured volume of the supplied fluid reaches a known volume of the cavity and the lumen to be purged, and due to the fact that air bubbles are able to exit the cavity and lumen at all times because of the correct orientation, a conclusion can be drawn that de-airing is complete. The system may then notify the user that purging is complete. It will be appreciated that a certain safety margin, i.e. additional purge fluid volume can be added until the system indicates a successful completion of the purging process. In addition, the user may be requested to confirm that the purge fluid exits the catheter at the distal end to complete the purging process.

As explained above, there may be no problems of purging the lumen of the elongate tubular portion of the catheter, while purging the at least one cavity of a connected device may be difficult.
In another preferred embodiment, the at least one sensor is arranged in the connected device of the catheter in order to detect its orientation. It may not be necessary to detect the orientation of the elongate tubular portion of the catheter.

The at least one sensor to detect the orientation of the respective portion of the catheter, in particular the orientation of at least one connected device, may be at least one of a gravity sensor, an accelerometer and a gyroscope. For the sake of convenience, any of these terms may refer to all of these terms. However, the term "sensor" in the sense of the present disclosure may include any other type of active or passive sensor, means or system which is able to detect an orientation of the respective portion of the catheter in three-dimensional space. These may include sensors which are based on a magnetic or electromagnetic field, optical sensors or the like.
For instance in another embodiment, the at least one sensor may be arranged at a distance to the catheter. The sensor may be formed by a camera system which records the respective portion of the catheter, in particular the orientation of at least one connected device, and detects the orientation from the recorded image.

However, a sensor is preferred which is easy to implement in existing systems and which provides reliable results. For instance, the advantage of a gravity sensor or accelerometer is that it does not require any external infrastructure, like external sensors or external field generators. In addition, an accelerometer is robust against external perturbations, such as a magnetic field, which may be caused for instance by an electric motoric of an intravascular blood pump. An accelerometer may be designed as a MEMS device or nanodevice and can be arranged on a printed circuit board (PCB) or printed circuit assembly (PCA) which already exists for example in a drive unit of an intravascular blood pump.

A gravity sensor or accelerometer may detect a "downward" direction, more specifically in a vertical direction. This "downward" direction, which represents the detected orientation, may be transferred to the control unit using memory-mapped register technique. That means, the sensor data, i.e. the detected orientation (downward direction) is stored in a memory, e.g. a memory of the blood pump. Every time the control unit reads a specific byte of the pump memory, the control unit gets the detected orientation, e.g. the tilt in degrees, instead of the memory content. That means, the accelerometer does not need an additional connection, e.g. wires, to the control unit to transfer the data of the sensor but uses the existing communication connection that reads the memory.

As mentioned above, the system and method according to the present invention may be particularly advantageous when used in connection with an intravascular blood pump, in particular an intravascular blood pump with a pump unit connected to an elongate body of the catheter. In this type of intravascular blood pumps, the interior of the catheter is in fluid communication with the environment and is purged during operation of the pump and has to be completely de-aired before use in order to avoid introducing air bubbles into the patient's vascularity. By detecting the orientation of the at least one connected device and guiding a user to correctly orient the catheter, especially at least one of the connected devices, the respective cavities can be securely de-aired. The pump unit may be expandable, i.e. it may be compressed into a compressed configuration for insertion into the blood vessel, and may be released and expanded once placed at the target position as will be appreciated by a person skilled in the art.

### BRIEF DESCRIPTION OF THE DRAWINGS

The foregoing summary, as well as the following detailed description of preferred embodiments, will be better understood if read in conjunction with the appended drawings. For the purpose of illustrating the present disclosure, reference is made to the drawings, which are to be understood as schematic and may not be true to scale. The drawings are exemplary for illustrating the present invention, and the scope of the disclosure is not limited to the specific embodiments disclosed in the drawings. In the drawings:
FIG. 1 shows a system in accordance with an embodiment of the present invention, including an intravascular blood pump connected to a control unit.
FIG. 2 shows the blood pump of FIG. 1 in more detail.
FIG. 3a to FIG. 3c schematically illustrate a handle portion or a pump unit of a catheter at different orientations during the purging process.

### DETAILED DESCRIPTION

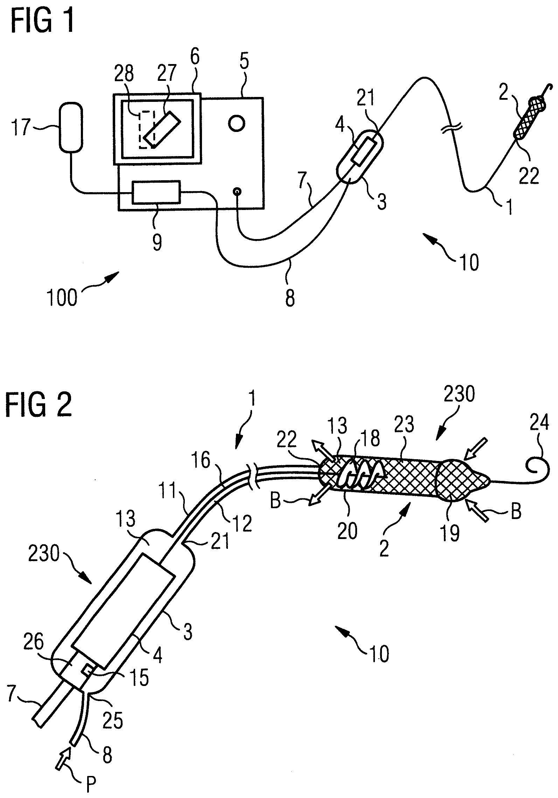

FIG. 1 shows a system 100 in accordance with an embodiment of the present invention. In particular, the system 100 comprises an intravascular blood pump 10 connected to a control unit 5. The intravascular blood pump 10 comprises a catheter 1 with a pump unit 2 connected to the distal end 22 of the catheter 1, more specifically to an elongate tubular portion 11 of the catheter 1 (see FIG. 2). The pump unit 2 is also called a connected device 230. A handle portion 3 is connected to a proximal end 21 of the elongate tubular portion 11. The handle portion 11 is also called a connected device 230. The handle portion 3 can be grabbed by a user for handling the catheter 1 and will not be inserted into the patient. The intravascular blood pump 10 may be designed to act as a left ventricular assist device and may be inserted into a patient's aorta toward the heart, more specifically the left ventricle of the patient's heart. In this case, the pump unit 2 will be placed through the aortic valve to convey blood from the left ventricle into the aorta. However, it will be appreciated that the blood pump 10 may be designed for a different application, such as to act as a right ventricular assist device, or the catheter may be designed for a different field than intravascular blood pumps.

Still referring to FIG. 1, the handle portion 3 accommodates a drive unit 4 of the blood pump 10, which will be explained in more detail below. In order to control the drive unit 4, an electric connecting cable 7 is provided connecting the intravascular blood pump 10 to the control unit 5. The control unit 5 has a user interface comprising a display 6. It will be appreciated that the control unit 5 may comprise various further control elements, such as a touch screen, control dials, buttons or the like. The display 6 may be configured to display various operation parameters of the blood pump 10, such as pump flow rate, pump speed, motor current, etc. In particular, in view of the present invention, the display 6 may display during an initial purging process a representation of the orientation of a connected device 230, for example the handle portion 3, which is detected by a sensor 15. As indicated in FIG. 1, the current orientation 27 of the connected device 230 may be displayed relative to a predetermined target orientation 28 of the connected device (indicated in dashed lines in FIG. 1). Any other display options suitable to guide a user may be implemented. Further, or in an alternative example, the orientation 27 of the pump unit 2 may be displayed.

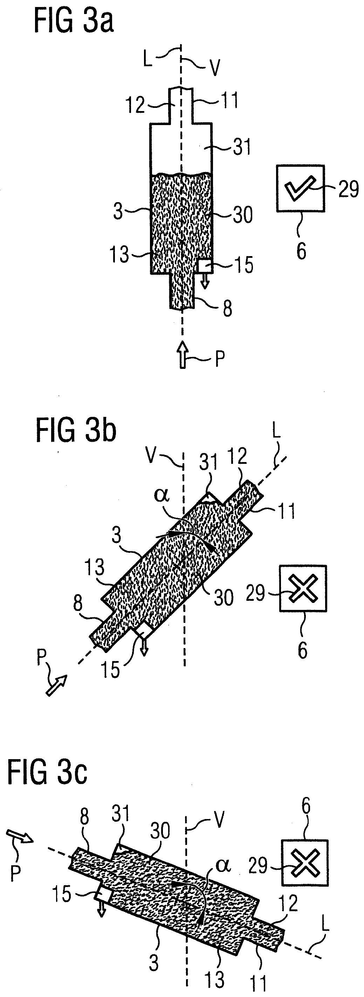

Besides the electrical connection, the blood pump 10 is further connected to a purge line 8 which is provided for supplying a purge fluid to the blood pump 10 for an initial purging and de-airing process as well as during operation of the blood pump 10. The purge line 8 may be connected to a purge cassette 9, which may be provided for generating a certain purge pressure, which may be for instance 500 mmHg. A purge fluid, which may be any suitable fluid, such as water, or preferably a dextrose solution, may be provided in a container or bag 17 and supplied to the purge cassette 9 and further, via the purge line 8, to the blood pump 10. Details of the method of preparing the blood pump 10 will be described in more detail below with respect to FIG. 3a to FIG. 3c.

Referring now to FIG. 2, the intravascular blood pump 10 of the system 100 of FIG. 1 is described in more detail. The blood pump 10 comprises two connected devices 230 which are the handle portion 3 and the pump unit 2. Each of the connected devices 230 comprises a cavity 13. Each of these cavities 13 is in fluid communication with a lumen 12 of the elongate tubular body 11. The blood pump 10 of the embodiment shown is an expandable catheter pump, in other words, the pump unit 2 is expandable from a compressed configuration (not shown) to an expanded configuration. More specifically, in order to insert the blood pump 10 through a small access into a patient's blood vessel, the pump unit 2 will be compressed to the compressed configuration to reduce its diameter. Once placed at a target location, for instance the left ventricle and the aorta, the pump unit 2 may expand. For this purpose, the pump unit 2 may comprise an expandable pump casing 23, which may be in the form of a cannula and may be formed of a mesh-like support structure covered by a thin polymer membrane. The support structure may be made of a shape memory material, such as nitinol. The pump unit 2 may have at its distal end an atraumatic tip 24, also known as J-tip or pig tail. An impeller 18, which may be expandable and compressible, respectively, as well, is disposed inside the pump casing 23. In particular in the aforementioned application for left ventricular support, a blood flow inlet 19 may be formed in a distal portion of the pump casing 23, for instance in an enlarged diameter portion. Blood enters the pump casing 23 and is conveyed by means of an impeller 18 rotating about an axis of rotation through the pump casing 23, and out of a blood flow outlet 20 (indicated by arrows B).

The impeller 18 is coupled to a flexible drive shaft 16, which extends through the catheter 1, more specifically through the lumen 12 of the elongate tubular body 11 of the catheter 1. The flexible drive shaft 16 is coupled to a drive unit 4 which is located inside the cavity 13 of the handle portion 3. The drive unit 4, which is only schematically illustrated in FIG. 2, may comprise an electric motor. Further electronic parts of the drive unit 4, such as a PCB 26, are connected to the cable 7, which in turn is connected to the control unit 5 as explained above with respect to FIG. 1.

A sensor 15 for detecting the orientation of the handle portion 3 is placed on the PCB 26 to be able to transmit data relating to the current orientation to the control unit 4. Before use in the patient, in particular the cavity 13 of the handle portion 3 and the lumen 12 of the elongated tubular portion 11 of the catheter 1, and also the pump unit 2, have to be completely de-aired, that means air bubbles have to be completely removed from all cavities of the blood pump 10 to prevent air from being introduced into the patient's vascularity, which may cause severe complications, such as an infarct. In order to supply a purge fluid to the blood pump 10, the purge line 8 is connected to a purge fluid inlet 25 of the handle portion 3. A person skilled in the art will understand that various connectors, such as Y-connectors, Luer-connectors or the like may be used to connect the various lines and cables. Further or in the alternative, a sensor may be placed in the pump unit, additionally or alone.

Referring now to FIG. 3a to FIG. 3c, a method of preparing a catheter in accordance with an embodiment of the present invention is described. In particular, as explained above, during the preparation of catheter 1 before it can be used in a patient, air bubbles shall be completely removed. While there is substantially no risk that air bubbles are trapped in the lumen 12 of the elongate tubular body 11 of the catheter 1, air bubbles might be trapped inside the cavity 13 of connected devices as the handle portion 3 and/or the pump unit 2. In particular, there is a risk that purge fluid exiting the catheter 1 at the distal end 22 (i.e. exiting the pump unit 2) may erroneously imply that the catheter 1 has been successfully de-aired. This is because the cavity 13 of the handle portion 3 (or the pump unit - not shown) may have recesses or other geometrical structures of the inner wall or of built-in components which may still be filled with air while the purge fluid already flows through the lumen 12 of the elongate tubular portion 11 of the catheter 1. In particular, the lumen 12 may have a very small diameter such that capillary forces may draw the purge fluid into the lumen 12 although there still is air in the cavity 13 of the handle portion 3 and/or the pump unit. Furthermore, the cavities or built-in components may have irregular structures that further increase the risk of entrapped air bubbles.

The risk of air bubbles being trapped inside the handle portion 3 and/or the pump unit 2 can be substantially reduced or eliminated if the handle portion 3 and/or the pump unit 2 is held in a correct orientation during the initial purge process for de-airing the catheter 1. Therefore, the system 100 of the present invention includes a sensor 15 for detecting the orientation of the handle portion 3 and/or the pump unit 2. In other words, the current orientation of the handle portion 3 and/or the pump unit 2 can be detected while supplying the purge fluid through the line 8 (see arrow P) into the handle portion 3 or into the pump unit via the tubular portion 11. In particular, the current orientation may be detected continuously or at certain time intervals, preferably very short time intervals, such that the orientation can be properly monitored.

The sensor 15 may be a gravity sensor, accelerometer, gyroscope or any other sensor, which is able to detect an orientation. In particular, it is sufficient to detect the orientation, and it may not be necessary to detect the absolute position of the handle portion 3 and/or the pump unit 2 in three-dimensional space, particularly in relation to gravity. Especially an accelerometer does not require any external infrastructure, like external field generators or the like, and is robust against a magnetic field, which may be caused by the drive unit 4. Thus, an accelerometer may provide an exact orientation.

In particular, the orientation may be represented by a tilt angle α enclosed by a general longitudinal axis L and/or the pump unit 2 and a vertical axis V, preferably along gravity. As indicated by the arrows in FIG. 3a to FIG. 3c, an accelerometer 15 typically detects a vertically downward direction. The sensor data can then be used to calculate the tilt angle α relative to the particular downward direction. The detected orientation may be transmitted to the control unit 5. Preferably, the sensor data obtained by the sensor 15 are transmitted using memory mapping, which eliminates the need for further connection wires, and the existing connecting cable 7 can also be used for transmitting the sensor data. The control unit 5 can display the detected orientation calculated from the sensor data on a display 6 as illustrated in FIG. 1. Further, the control unit 5 may be configured to guide the user to change the orientation of the handle portion 3 or the pump unit 2 toward a predetermined target orientation. As illustrated in FIG. 3a to FIG. 3c, the display 6 may also give a positive and negative indication 29 whether the current orientation matches the predetermined target orientation or not, respectively.

A de-airing process will be described with the handle in FIG. 3a-3c as an example that can also be applied to the pump unit 2 analogously: The predetermined target orientation may be chosen depending on the interior geometry of the handle portion 3, to be the orientation in which air bubbles can securely exit the cavity 13 of the handle portion 3 into the lumen 12 of the elongated tubular portion 11 of the catheter 1. The predetermined target orientation may be a vertical orientation as shown in FIG. 3a, in which further a purge fluid line 8 is located vertically below the proximal end 21 of the tubular portion 11, i.e. at an angle α of 0 degrees, or, in other words, in which the vertical axis V is coincident with the longitudinal axis L. In this orientation, the purge fluid 30 is introduced from below and can securely push any air 31 in a vertically upward direction such that the cavity 13 and that the lumen 12 can be securely de-aired. It will be appreciated that any angle α greater than 0 or any range of angles may be selected to define the predetermined target orientation.

As shown in FIG. 3b, if the handle portion 3 is held at an oblique angle, for instance an angle α greater than 0 degree and below 90 degrees, such as 45 degrees, air 31 may be trapped in a corner of the cavity 13 of the handle portion 3 while the purge fluid 30 already is present in lumen 12. Likewise, as shown in FIG. 3c, if the handle portion 3 is held in an orientation in which the fluid inlet into the handle portion 3 is located vertically above the exit into the lumen 12, i.e. for instance at an angle α greater than 90 degrees up to 180 degrees, air 31 may be trapped in a corner of the cavity 13 of the handle portion 3. Nevertheless, purge fluid may continuously exit the catheter 1 at the distal end 22, thereby making the user erroneously believe that he has successfully de-aired the catheter 1.

Therefore, the user is guided how to hold the handle portion 3, in other words how to change the orientation of the handle portion 3 such that it matches the predetermined target orientation. The detected orientation, that means the current orientation detected by the sensor 15 is compared to the predetermined target orientation and a suitable indication is displayed on the display 6, e.g. at least one of the indications 27, 28 and 29 as described above. It will be appreciated that any indication representing the difference between the current detected orientation and the predetermined target orientation suitable to guide a user to choose the correct orientation may be envisioned, such as graphic illustrations, color scales, arrows or the like. Likewise, in addition or alternatively, an acoustic signal may be used to guide the user to change the correct orientation to approach and match the predetermined target orientation, which is necessary for completely de-airing the system.

While the purge fluid may be continuously supplied by the fluid line 8 to the cavity 13 and to the lumen 12 during the aforementioned purging and de-airing process, it may be advantageous to activate the flow of purge fluid only if the user holds the handle portion 3 in the correct orientation. Vice versa, the flow of purge fluid may be stopped or paused if the user does not correctly hold the handle portion 3. This may reduce or eliminate the risk that the purge fluid is drawn into the lumen 12 too early although air is still present in the handle portion 3. The volume of supplied purge fluid may be measured such that based on the known volume of all cavities in the catheter 1, an indication may be displayed once the purging and de-airing process is completed. For safety reasons, the control unit 5 may prompt the user to confirm that the purge fluid exits the blood pump 10 at the distal end, and a safety margin for the amount of purge fluid may be added.

The disclosed method and system allow for proper and secure preparation of a catheter for use in a patient, in particular proper purging and de-airing the catheter in order to prevent air bubbles from being introduced into the patient's vascularity. It will be appreciated that the invention is particularly useful in the field of intravascular blood pumps but may be implemented in any catheter which requires proper de-airing before used in a patient.

## Claims

1. A system (100), comprising a catheter (1), the catheter (1) comprising an elongate tubular portion (11) and at least one connected device (230), the elongate tubular portion (11) configured to be inserted into a patient's blood vessel and having a proximal end (21) and a distal end (22) and a lumen (12) extending from the proximal end (21) to the distal end (22), each connected device (230) connected to the elongate tubular portion (11) and having at least one cavity (13), wherein the at least one cavity (13) is in fluid communication with the lumen (12) of the elongate tubular portion (11),
wherein the system further comprises at least one sensor (15) for detecting an orientation of at least one of the elongate tubular portion (11) and the at least one connected device (230), **characterized in that** the system further comprises a control unit (5) configured to receive data from the at least one sensor (15) including the orientation detected by the at least one sensor (15) and to determine whether the detected orientation matches an orientation in which air is releasable from the cavity (13), wherein the control unit (5) is configured to compute a difference of the detected orientation and a predetermined orientation to guide a user to change the orientation in order to approach the predetermined orientation.

2. The system of claim 1, wherein the control unit (5) is configured to display at least one of: a matching indicator representing the matching of a detected orientation with an orientation in which air is releasable from the cavity (13), the detected orientation, and a representation indicating the difference between the detected orientation and the predetermined orientation.

3. The system of claim 1 or 2, wherein the predetermined orientation is a vertical orientation or a conical range around a vertical axis defined by a predetermined angle.

4. The system of any one of claims 1 to 3, wherein the at least one sensor (15) is arranged in the connected device (230) and configured to detect an orientation of the connected device (230).

5. The system of any one of claims 1 to 4, wherein the at least one sensor (15) includes at least one of a gravity sensor, an accelerometer and a gyroscope.

6. The system of any one of claims 1 to 5, further comprising a pump unit (2), and a drive unit (4) to provide an intravascular blood pump (10) for percutaneous insertion into a patient's blood vessel, wherein the pump unit (2) is one of the at least one connected device (230) and is disposed at the distal end (22) of the elongate tubular portion (11) of the catheter (1) and comprises a cavity (13), the pump unit (2) including an impeller (18) which is rotatable about an axis of rotation to convey blood from a blood flow inlet (19) to a blood flow outlet (20) of the pump unit (2), wherein the pump unit (2) is preferably an expandable pump unit with the impeller (18) being coupled to a flexible drive shaft (16) which extends through the lumen (12) of the elongate tubular portion (11) of the catheter (1), and wherein the drive unit (4) is disposed in the at least one cavity (13) of a handle portion (3) which is another one of the at least one connected device (230) and which is coupled to the flexible drive shaft (16) so as to cause rotation of the impeller (18).

7. A method of preparing a catheter (1) for use in a patient, the catheter (1) comprising an elongate tubular portion (11) and a at least one connected device (230), the elongate tubular portion (11) being configured to be inserted into a patient's blood vessel and having a proximal end (21) and a distal end (22) and a lumen (12) extending from the proximal end (21) to the distal end (22), each of the at least one connected device (230) connected to the elongate tubular portion (11) and having at least one cavity (13), wherein the at least one cavity (13) is in fluid communication with the lumen (12) of the elongate tubular portion (11),
the method comprising the steps of:
- supplying a fluid (30) into at least one of (a) the at least one cavity (13) of at least one of the at least one connected device (230) and (b) the lumen (12) of the elongate tubular portion (11) to purge at least one of the at least one cavity (13) and the lumen (12); and
- detecting an orientation of at least one of the elongate tubular portion (11) and the at least one connected device (230),
**characterized in that** the method further comprises the steps of:
- determining whether the detected orientation matches an orientation in which air is releasable from the cavity (13); and
- computing a difference between the detected orientation and a predetermined orientation to guide a user to change the orientation in order to approach the predetermined orientation.

8. The method of claim 7, further comprising displaying at least one of: a matching indicator representing the matching of a detected orientation with an orientation in which air is releasable from the cavity (13), the detected orientation, and a representation indicating a difference between the detected orientation and the predetermined orientation.

9. The method of claim 7 or 8, wherein the predetermined orientation is a vertical orientation or a conical range around a vertical axis defined by a predetermined angle.

10. The method of any one claims 7 to 9, wherein the fluid supply is activated, when the detected orientation matches the predetermined orientation, and the fluid supply is deactivated, when the detected orientation differs from the predetermined orientation.

11. The method of any one of claims 7 to 10, further comprising measuring a volume of the supplied fluid (30).

12. The method of any one of claims 7 to 11, wherein the orientation is detected by means of at least one sensor (15), wherein the at least one sensor (15) includes at least one of a gravity sensor, an accelerometer and a gyroscope, wherein the at least one sensor (15) is preferably arranged in at least one of a handle portion (3) and a pump unit (2) and the elongate tubular portion (11), wherein the handle portion (3) is one of the at least one connected device (230) and the pump unit (2) is another one of the at least one connected device (230), wherein the sensor preferably includes a camera that is preferably arranged outside of the handle portion (3) and the pump unit (2) and the elongate tubular portion (11).

## Patentansprüche

1. System (100), das einen Katheter (1) aufweist, wobei
der Katheter (1) einen länglichen rohrförmigen Abschnitt (11) und mindestens eine angeschlossene Vorrichtung (230) umfasst, wobei
der längliche rohrförmige Abschnitt (11) so konfiguriert ist, dass er in ein Blutgefäß eines Patienten eingeführt werden kann, und ein proximales Ende (21) und ein distales Ende (22) und ein Lumen (12) aufweist, das sich von dem proximalen Ende (21) zu dem distalen Ende (22) erstreckt, wobei
jede angeschlossene Vorrichtung (230) mit dem länglichen rohrförmigen Abschnitt (11) verbunden ist und mindestens einen Hohlraum (13) aufweist, wobei
der mindestens eine Hohlraum (13) in Fluidverbindung mit dem Lumen (12) des länglichen rohrförmigen Abschnitts (11) steht, wobei
das System ferner mindestens einen Sensor (15) zum Erfassen von mindestens einer der Ausrichtungen des länglichen rohrförmigen Abschnitts (11) und der mindestens einen angeschlossenen Vorrichtung (230) umfasst,
**dadurch gekennzeichnet, dass**
das System ferner eine Steuereinheit (5) umfasst, die dazu ausgebildet ist, dass die Steuereinheit (5) Daten von dem mindestens einem Sensor (15) einschließlich der von dem mindestens einen Sensor (15) erfassten Ausrichtung empfängt und bestimmt, ob die erfasste Ausrichtung mit einer Ausrichtung übereinstimmt, in der Luft aus dem Hohlraum (13) freigesetzt werden kann, wobei
die Steuereinheit (5) dazu ausgebildet ist, dass die Steuereinheit (5) eine Differenz zwischen der erfassten Ausrichtung und einer vorbestimmten Ausrichtung berechnet, um einen Benutzer anzuleiten, die Ausrichtung zu ändern, um sie der vorbestimmten Ausrichtung anzunähern.

2. System nach Anspruch 1, wobei die Steuereinheit (5) dazu ausgebildet ist, dass die Steuereinheit (5) mindestens eines von Folgendem anzeigt:
einen Übereinstimmungsindikator, der die Übereinstimmung einer erfassten Ausrichtung mit einer Ausrichtung darstellt, in der Luft aus dem Hohlraum (13) freigesetzt werden kann, die erfasste Ausrichtung und eine Darstellung, die die Differenz zwischen der erfassten Ausrichtung und der vorbestimmten Ausrichtung anzeigt.

3. System nach Anspruch 1 oder 2, wobei die vorbestimmte Ausrichtung eine vertikale Ausrichtung oder ein konischer Bereich um eine durch einen vorbestimmten Winkel definierte vertikale Achse ist.

4. System nach einem der Ansprüche 1 bis 3, wobei der mindestens eine Sensor (15) in der angeschlossenen Vorrichtung (230) angeordnet und derart ausgebildet ist, dass der mindestens eine Sensor (15) eine Ausrichtung der angeschlossenen Vorrichtung (230) erfasst.

5. System nach einem der Ansprüche 1 bis 4, wobei der mindestens eine Sensor (15) mindestens einen Schwerkraftsensor, einen Beschleunigungsmesser oder ein Gyroskop umfasst.

6. System nach einem der Ansprüche 1 bis 5, das ferner eine Pumpeneinheit (2) und eine Antriebseinheit (4) umfasst, um eine intravaskuläre Blutpumpe (10) zur perkutanen Einführung in ein Blutgefäß eines Patienten bereitzustellen, wobei die Pumpeneinheit (2) eine der mindestens einen angeschlossenen Vorrichtung (230) ist und am distalen Ende (22) des länglichen rohrförmigen Abschnitts (11) des Katheters (1) angeordnet ist und einen Hohlraum (13) umfasst, wobei die Pumpeneinheit (2) ein Flügelrad (18) umfasst, das um eine Drehachse drehbar ist, um Blut von einem Blutstromeinlass (19) zu einem Blutstromauslass (20) der Pumpeneinheit (2) zu fördern, wobei die Pumpeneinheit (2) vorzugsweise eine expandierbare Pumpeneinheit ist, wobei das Laufrad (18) mit einer flexiblen Antriebswelle (16) gekoppelt ist, die sich durch das Lumen (12) des länglichen rohrförmigen Abschnitts (11) des Katheters (1) erstreckt, und wobei die Antriebseinheit (4) in dem mindestens einen Hohlraum (13) eines Griffabschnitts (3) angeordnet ist, der eine weitere der mindestens einen angeschlossenen Vorrichtung (230) ist und der mit der flexiblen Antriebswelle (16) gekoppelt ist, um eine Drehung des Laufrads (18) zu bewirken.

7. Verfahren zum Vorbereiten eines Katheters (1) zur Verwendung in einem Patienten, wobei der Katheter (1) einen länglichen rohrförmigen Abschnitt (11) und mindestens eine angeschlossene Vorrichtung (230) umfasst, wobei der längliche rohrförmige Abschnitt (11) derart ausgebildet ist, dass er in ein Blutgefäß eines Patienten eingeführt werden kann, und ein proximales Ende (21) und ein distales Ende (22) und ein Lumen (12) aufweist, das sich von dem proximalen Ende (21) zu dem distalen Ende (22) erstreckt, wobei jede der mindestens einen angeschlossenen Vorrichtung (230) mit dem länglichen rohrförmigen Abschnitt (11) verbunden ist und mindestens einen Hohlraum (13) aufweist, wobei der mindestens eine Hohlraum (13) in Fluidverbindung mit dem Lumen (12) des länglichen rohrförmigen Abschnitts (11) steht, wobei das Verfahren die folgenden Schritte umfasst:
- Zuführen eines Fluides (30) in (a) den mindestens einen Hohlraum (13) mindestens einer der mindestens einen angeschlossenen Vorrichtung (230) und/oder (b) das Lumen (12) des länglichen rohrförmigen Abschnitts (11), um den mindestens einen Hohlraum (13) und/oder das Lumen (12) zu spülen; und
- Erfassen einer Ausrichtung des länglichen rohrförmigen Abschnitts (11) und/oder der mindestens einen angeschlossenen Vorrichtung (230),
**dadurch gekennzeichnet, dass** das Verfahren ferner die folgenden Schritte umfasst:
- Bestimmen, ob die erfasste Ausrichtung mit einer Ausrichtung übereinstimmt, in der Luft aus dem Hohlraum (13) freigesetzt werden kann; und
- Berechnen einer Differenz zwischen der erfassten Ausrichtung und einer vorbestimmten Ausrichtung, um einen Benutzer anzuleiten, die Ausrichtung zu ändern, um sie der vorbestimmten Ausrichtung anzunähern.

8. Verfahren nach Anspruch 7, ferner umfassend das Anzeigen von mindestens einem von: einem Übereinstimmungsindikator, der die Übereinstimmung einer erfassten Ausrichtung mit einer Ausrichtung darstellt, in der Luft aus dem Hohlraum (13) freigesetzt werden kann, der erfassten Ausrichtung und einer Darstellung, die eine Differenz zwischen der erfassten Ausrichtung und der vorbestimmten Ausrichtung anzeigt.

9. Verfahren nach Anspruch 7 oder 8, wobei die vorbestimmte Ausrichtung eine vertikale Ausrichtung oder ein konischer Bereich um eine vertikale Achse ist, der durch einen vorbestimmten Winkel definiert ist.

10. Verfahren nach einem der Ansprüche 7 bis 9, wobei die Fluidzuführung aktiviert wird, wenn die erfasste Ausrichtung mit der vorbestimmten Ausrichtung übereinstimmt, und die Fluidzuführung deaktiviert wird, wenn die erfasste Ausrichtung von der vorbestimmten Ausrichtung abweicht.

11. Verfahren nach einem der Ansprüche 7 bis 10, ferner umfassend das Messen eines Volumens des zugeführten Fluides (30).

12. Verfahren nach einem der Ansprüche 7 bis 11, wobei die Ausrichtung mittels mindestens eines Sensors (15) erfasst wird, wobei der mindestens eine Sensor (15) mindestens einen von einem Schwerkraftsensor, einem Beschleunigungsmesser und einem Gyroskop umfasst, wobei der mindestens eine Sensor (15) vorzugsweise in mindestens einem von einem Griffabschnitt (3) und einer Pumpeneinheit (2) und dem länglichen rohrförmigen Abschnitt (11) angeordnet ist, wobei der Griffabschnitt (3) eine der mindestens einen angeschlossenen Vorrichtung (230) ist und die Pumpeneinheit (2) eine andere der mindestens einen angeschlossenen Vorrichtung (230) ist, wobei der Sensor vorzugsweise eine Kamera umfasst, die vorzugsweise außerhalb des Griffabschnitts (3) und der Pumpeneinheit (2) und des länglichen rohrförmigen Abschnitts (11) angeordnet ist

## Revendications

1. Système (100), comprenant un cathéter (1), le cathéter (1) comprenant une portion tubulaire oblongue (11) et au moins un dispositif connecté (230), la portion tubulaire oblongue (11) étant configurée pour être introduite dans un vaisseau sanguin d'un patient et présentant une extrémité proximale (21) et une extrémité distale (22) et une lumière (12) s'étendant de l'extrémité proximale (21) à l'extrémité distale (22), chaque dispositif connecté (230) étant connecté à la portion tubulaire oblongue (11) et présentant au moins une cavité (13), dans lequel la au moins une cavité (13) est en communication de fluide avec la lumière (12) de la portion tubulaire oblongue (11),
dans lequel le système comprend en outre au moins un capteur (15) pour détecter une orientation d'au moins un élément parmi la portion tubulaire oblongue (11) et le au moins un dispositif connecté (230), **caractérisé en ce que** le système comprend en outre une unité de commande (5) configurée pour recevoir des données du au moins un capteur (15) incluant l'orientation détectée par le au moins un capteur (15) et pour déterminer si oui ou non l'orientation détectée concorde avec une orientation où l'air peut être libéré de la cavité (13), et dans lequel l'unité de commande (5) est configurée pour calculer une différence de l'orientation détectée et une orientation prédéterminée pour guider un utilisateur pour modifier l'orientation afin de se rapprocher de l'orientation prédéterminée.

2. Système selon la revendication 1, dans lequel l'unité de commande (5) est configurée pour afficher au moins un des éléments suivants : un indicateur de concordance représentant la concordance d'une orientation détectée avec une orientation où l'air peut être libéré de la cavité (13), l'orientation détectée, et une représentation indiquant la différence entre l'orientation détectée et l'orientation prédéterminée.

3. Système selon la revendication 1 ou 2, dans lequel l'orientation prédéterminée est une orientation verticale ou une plage conique autour d'un axe vertical défini par un angle prédéterminé.

4. Système selon l'une quelconque des revendications 1 à 3, dans lequel le au moins un capteur (15) est agencé dans le dispositif connecté (230) et configuré pour détecter une orientation du dispositif connecté (230).

5. Système selon l'une quelconque des revendications 1 à 4, dans lequel le au moins un capteur (15) inclut au moins un élément parmi un détecteur de gravité, un accéléromètre, et un gyroscope.

6. Système selon l'une quelconque des revendications 1 à 5, comprenant en outre une unité formant pompe (2) et une unité d'entraînement (4) pour fournir une pompe sanguine intravasculaire (10) pour une introduction percutanée dans un vaisseau sanguin d'un patient, dans lequel l'unité formant pompe (2) est un des au moins un dispositif connecté (230), et est disposée sur l'extrémité distale (22) de la portion tubulaire oblongue (11) du cathéter (1), et comprend une cavité (13), l'unité formant pompe (2) incluant un impulseur (18), lequel est rotatif autour d'un axe de rotation afin d'acheminer du sang d'une admission de flux sanguin (19) vers une sortie de flux sanguin (20) de l'unité formant pompe (2) ; dans lequel l'unité formant pompe (2) est de préférence une unité formant pompe extensible, l'impulseur (18) étant couplé avec un arbre d'entraînement flexible (16), lequel s'étend à travers la lumière (12) de la portion tubulaire oblongue (11) du cathéter (1), et dans lequel l'unité d'entraînement (4) est disposée dans la au moins une cavité (13) d'une portion de poignée (3), laquelle est un autre dispositif parmi le au moins un dispositif connecté (230) et est couplée à l'arbre d'entraînement flexible (16) de manière à provoquer la rotation de l'impulseur (18).

7. Procédé destiné à préparer un cathéter (1) à utiliser dans un patient, le cathéter (1) comprenant une portion tubulaire oblongue (11) et au moins un dispositif connecté (230), la portion tubulaire oblongue (11) étant configurée pour être introduite dans un vaisseau sanguin d'un patient et présentant une extrémité proximale (21) et une extrémité distale (22) et une lumière (12) s'étendant de l'extrémité proximale (21) à l'extrémité distale (22), chacun des au moins un dispositif connecté (230) étant connecté à la portion tubulaire oblongue (11) et présentant au moins une cavité (13), dans lequel la au moins une cavité (13) est en communication de fluide avec la lumière (12) de la portion tubulaire oblongue (11),
le procédé comprenant les étapes suivantes :
- alimenter un fluide (30) dans au moins un des éléments parmi (a) la au moins une cavité (13) d'au moins un des au moins un dispositif connecté (230), et (b) la lumière (12) de la portion tubulaire oblongue (11), afin de purger au moins un élément parmi la au moins une cavité (13) et la lumière (12), et
- détecter une orientation d'au moins un élément parmi la portion tubulaire oblongue (11) et le au moins un dispositif connecté (230),
**caractérisé en ce que** le procédé comprend en outre les étapes suivantes :
- déterminer si oui ou non l'orientation détectée concorde avec l'orientation où l'air peut être libéré de la cavité (13), et
- calculer une différence entre l'orientation détectée et une orientation prédéterminée afin de guider un utilisateur pour modifier l'orientation afin de se rapprocher de l'orientation prédéterminée.

8. Procédé selon la revendication 7, comprenant en outre l'étape pour afficher au moins un des éléments suivants : un indicateur de concordance représentant la concordance d'une orientation détectée avec une orientation où l'air peut être libéré de la cavité (13), l'orientation détectée, et une représentation indiquant une différence entre l'orientation détectée et l'orientation prédéterminée.

9. Procédé selon la revendication 7 ou 8, dans lequel l'orientation prédéterminée est une orientation verticale ou une plage conique autour d'un axe vertical défini par un angle prédéterminé.

10. Procédé selon l'une quelconque des revendications 7 à 9, dans lequel l'alimentation en fluide est activée, lorsque l'orientation détectée concorde avec l'orientation prédéterminée, et l'alimentation en fluide est désactivée, lorsque l'orientation détectée est différente de l'orientation prédéterminée.

11. Procédé selon l'une quelconque des revendications 7 à 10, comprenant en outre l'étape pour mesurer un volume du fluide alimenté (30).

12. Procédé selon l'une quelconque des revendications 7 à 11, dans lequel l'orientation est détectée à l'aide d'au moins un capteur (15), dans lequel le au moins un capteur (15) inclut au moins un élément parmi un détecteur de gravité, un accéléromètre, et un gyroscope ; dans lequel le au moins un capteur (15) est de préférence agencé dans au moins un élément parmi une portion de poignée (3) et une unité formant pompe (2) et la portion tubulaire oblongue (11) ; dans lequel la portion de poignée (3) est au moins un élément parmi le au moins un dispositif connecté (230), et l'unité formant pompe (2) est un autre élément parmi le au moins un dispositif connecté (230), et dans lequel le capteur inclut de préférence une caméra, laquelle est de préférence agencée hors de la portion de poignée (3), de l'unité formant pompe (2), et de la portion tubulaire oblongue (11).
